# EUROPÄISCHE PATENTANMELDUNG

(11) **EP 0 770 595 A1**
(43) Veröffentlichungstag der Anmeldung: **02.05.1997**
(21) Anmeldenummer: 96116674.1
(22) Anmeldetag: 17.10.1996
(51) Int. Cl.: C07C 217/08, C07C 219/06, C07C 219/08, C11D 1/62, A61K 7/06, A61K 7/48, A61K 7/50

(54) **Sorbitolesterquats und ihre Verwendung zur Herstellung von oberflächenaktiven Mitteln, insbesondere Mitteln zur Haar- und Körperpflege**

(30) Priorität: 26.10.1995 DE 19539876
(71) Anmelder: Henkel Kommanditgesellschaft auf Aktien, 40589 Düsseldorf-Holthausen (DE)
(72) Erfinder: Pi Subirana, Rafael, Dr., 08400 Granollers (ES); Bonastre, Nuria, Dr., 080210 Barbéra del Vallés (ES); Prat Queralt, Ester, Dr., 08238 Galella, Barcelona (ES); Bigorra Llosas, Joaquim, Dr., 08203 Sabadell (ES)

(57) **Zusammenfassung**

Es werden neue Esterquats auf Zuckerbasis vorgeschlagen, die man erhält, indem man Trialkanolamine mit einer Mischung aus Fettsäuren, Dicarbonsäuren und Sorbitol umsetzt, die resultierenden Ester gegebenenfalls alkoxyliert und anschließend in an sich bekannter Weise quaterniert. Es werden kationische Tenside erhalten, die eine besonders hohe ökotoxikologische Verträglichkeit besitzen, synthetischen wie natürlichen Fasern einen angenehmen Griff verleihen und dabei gleichzeitig die elektrostatische Aufladung zwischen den Filamenten herabsetzen.

## Beschreibung

### Gebiet der Erfindung

Die Erfindung betrifft neue zuckerbasierte Esterquats, die man erhält, indem man Trialkanolamine zusammen mit einer Mischung aus Fettsäuren, Dicarbonsäuren und Sorbitol umsetzt und die resultierenden Ester - gegebenenfalls nach Alkoxylierung - in an sich bekannter Weise quaterniert.

### Stand der Technik

Unter der Bezeichnung 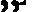 Esterquats 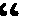 werden im allgemeinen quaternierte Fettsäuretriethanolaminestersalze verstanden die sich in breitem Umfang sowohl für die Faser- als auch für die Haaravivage eignen. In den vergangenen Jahren haben diese Stoffe infolge ihrer besseren ökotoxikologischen Verträglichkeit konventionelle quartäre Ammoniumverbindungen wie z.B. das bekannte Distearyldimethylammoniumchlorid zu einem guten Teil vom Markt verdrängt. Übersichten zu diesem Thema sind beispielsweise von O.Ponsati in **C.R. CED-Kongress, Barcelona, 1992, S.167 ,** R.Puchta et al. in **Tens.Surf.Det., 30, 186 (1993),** M.Brock in **Tens. Surf. Det. 30, 394 (1993)** und R.Lagerman et al. in **J.Am.Oil.Chem.Soc., 71, 97 (1994)** erschienen.

Kationische Avivagemittel auf Basis von Aminozuckern (Glucamin, Bis-Bissorbitylamin) sind ferner aus den Deutschen Offenlegungsschriften **DE-A1 42 38 207, DE 42 38 212, DE-A1 42 38 213, DE-A1 42 38 216** und **DE-A1 42 38 217** (Henkel) bekannt.

Obschon Esterquats des Stands der Technik über sehr gute anwendungstechnische Eigenschaften verfügen sowie eine zufriedenstellende biologische Abbaubarkeit und eine gute hautkosmetische Verträglichkeit besitzen, gehen die Anforderungen des Verbrauchers weiterhin in die Richtung verbesserter Produkteigenschaften.

Demzufolge hat die Aufgabe der Erfindung darin bestanden, neue Esterquat-Typen zur Verfügung zu stellen, die sich durch eine weiter verbesserte ökotoxikologische Verträglichkeit auszeichnen und gleichzeitig ein gutes Schaum-, Avivage- und Antistatikverhalten aufweisen.

### Beschreibung der Erfindung

Gegenstand der Erfindung sind Sorbitolesterquats, die man erhält, indem man Trialkanol-amine mit einer Mischung aus Fettsäuren, Dicarbonsäuren und Sorbitol umsetzt, die resul-tierenden Ester gegebenenfalls alkoxyliert und anschließend in an sich bekannter Weise qua-terniert.

Durch Veresterung von Trialkanolaminen mit einer Mischung von Fettsäuren, Dicarbonsäuren und Sorbitol werden neue zuckerbasierte Esterquats erhalten, die sich überraschenderweise gegenüber Produkten des Stands der Technik nicht nur durch eine besonders gute ökotoxikologische Verträglichkeit, sondern auch ausgezeichnete Haar- und sowie eine Verminde-rung der elektrostatischen Aufladung zwischen den Faserfilamenten auszeichnen.

Ein weiterer Gegenstand der Erfindung betrifft ein Verfahren zur Herstellung von Sorbitolesterquats, bei dem man Trialkanolamine mit einer Mischung aus Fettsäuren, Dicarbonsäuren und Sorbitol umsetzt und die resultierenden Ester gegebenenfalls nach Alkoxylierung anschließend in an sich bekannter Weise quaterniert.

### Trialkanolamine

Beispiele für Trialkanolamine, die im Sinne der Erfindung als zentrale Stickstoffverbindungen in Betracht kommen, sind in erster Linie Triethanolamin sowie Anlagerungsprodukte von 1 bis 10 und vorzugsweise 2 bis 5 Mol Ethylenoxid an diese Verbindungen.

### Fettsäuren

Unter Fettsäuren sind aliphatische Carbonsäuren der Formel **(I)** zu verstehen, 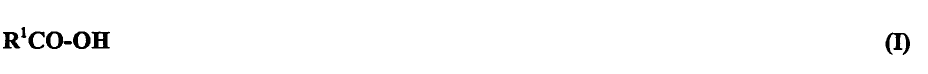 in der R¹CO für einen aliphatischen linearen oder verzweigten Acylrest mit 6 bis 22 Kohlenstoffatomen und 0 und/oder 1, 2 oder 3 Doppelbindungen steht. Typische Beispiele sind Capronsäure, Caprylsäure, 2-Ethylhexansäure, Caprinsäure, Laurinsäure, Isotridecan-säure, Myristinsäure, Palmitinsäure, Palmoleinsäure, Stearinsäure, Isostesrinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen wie beispielsweise Kokos-, Palm-, Palmkern- oder Talgfettsäure, vorzugsweise in gehärteter bzw. teilgehärteter Form.

### Dicarbonsäuren

Dicarbonsäuren, die im Sinne der Erfindung in Betracht kommen, folgen der Formel **(II)**, 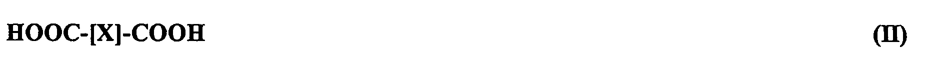 in der X für eine gegebenenfalls hydroxy- oder carboxysubstituierte Alkylengruppe mit 2 bis 12 Kohlenstoffatomen oder einen Phenylrest steht. Typische Beispiele sind daher Dicarbonsäuren wie beispielsweise Bernsteinsäure, Maleinsäure, Glutarsäure, Adipinsäure oder 1,12-Dodecandisäure, hydroxy- und/oder carboxyfunktionalisierte Dicarbonsäuren wie beispielsweise Weinsäure, Äpfelsäure oder Citronensäure oder aromatische Dicarbonsäuren wie beispielsweise die isomeren Phthalsäuren.

### Veresterung

Die Fettsäuren und die Dicarbonsäuren können im molaren Verhältnis von 1 : 10 bis 10 : 1 eingesetzt werden. Es hat sich jedoch als vorteilhaft erwiesen, ein molares Verhältnis von 4 : 1 bis 6 : 1 einzustellen. Die Trialkanolamine einerseits und die Säuren - also Fettsäuren und Dicarbonsäuren zusammengenommen - können im molaren Verhältnis 1 : 1,3 bis 1 : 2,4 eingesetzt werden. Als optimal hat sich ein molares Verhältnis Trialkanolamin : Säuren von 1 : 1,4 bis 1 : 1,8 erwiesen. Das molare Einsatzverhältnis Dicarbonsäuren : Sorbitol kann schließlich 1 : 0,75 bis 1 : 1,25 betragen.

Die Veresterung kann in an sich bekannter Weise durchgeführt werden, wie sie beispielsweise in der Internationalen Patentanmeldung **WO 91/01295** (Henkel) beschrieben wird. Vorteilhafterweise erfolgt die Veresterung bei Temperaturen von 120 bis 220 und insbesondere 130 bis 170°C und Drücken von 0,01 bis 1 bar. Als geeignete Katalysatoren haben sich hypophosphorige Säuren bzw. deren Alkalisalze, vorzugsweise Natriumhypophosphit bewährt, die in Mengen von 0,01 bis 0,1 und vorzugsweise 0,05 bis 0,07 Gew.-% - bezogen auf die Einsatzstoffe - eingesetzt werden können. Im Hinblick auf eine besonders hohe Farbqualität und -stabilität hat sich die Mitverwendung von Alkali- und/oder Erdalkaliborhydriden, wie beispielsweise Kalium-, Magnesium- und insbesondere Natriumborhydrid als vorteilhaft erwiesen. Die Co-Katalysatoren setzt man üblicherweise in Mengen von 50 bis 1000 und insbesondere 100 bis 500 ppm - wieder bezogen auf die Einsatzstoffe - ein. Entsprechende Verfahren sind auch Gegenstand der beiden Deutschen Patentschriften **DE-C1 43 08 792** und **DE-C1 44 09 322** (Henkel), auf deren Lehren hiermit ausdrücklich Bezug genommen wird.

Es ist möglich, Mischungen aus Fettsäuren, Dicarbonsäuren und Sorbitol einzusetzen oder aber die Veresterung in zwei Stufen durchzuführen, beispielsweise, indem man zunächst die Trialkanolamine mit einer Mischung aus Fettsäuren und Dicarbonsäuren partiell verestert und die abschließende Veresterung mit Sorbitol als zweiten Schritt durchführt oder aber, daß man die Trialkanolaminfettsäureester mit Dicarbonsäuremonosorbitolestern umsetzt.

### Alkoxylierung

Zur Herstellung von polyalkylenoxidhaltigen Sorbitolesterquats kann man nach zwei Alternativen verfahren. Zum einen kann man ethoxylierte Trialkanolamine einsetzen. Dies hat den Vorteil, daß die Alkylenoxidverteilung im später resultierenden Sorbitolesterquat bezüglich der drei OH-Gruppen des Amins annähernd gleich ist. Nachteilig ist jedoch, daß die Veresterung aus sterischen Gründen schwieriger wird. Die Methode der Wahl besteht daher darin, den Ester vor der Quaternierung zu alkoxylieren. Dies kann in an sich bekannter Weise geschehen, d.h. in Anwesenheit basischer Katalysatoren und bei erhöhten Temperaturen. Als Katalysatoren kommen beispielsweise Alkali- und Erdalkalihydroxide und -alkoholate, vorzugsweise Natriumhydroxid und insbesondere Natriummethanolat in Betracht; die Einsatzmenge liegt üblicherweise bei 0,5 bis 5 und vorzugsweise 1 bis 3 Gew.-% - bezogen auf die Einsatzstoffe. Bei Verwendung dieser Katalysatoren werden in erster Linie freie Hydroxylgruppen alkoxyliert. Setzt man als Katalysatoren jedoch calcinierte oder mit Fettsäuren hydrophobierte Hydrotalcite ein, kommt es auch zu einer Insertion der Alkylenoxide in die Esterbindungen. Diese Methode ist bevorzugt, wenn man eine Alkylenoxidverteilung wünscht, die der bei Einsatz von alkoxylierten Trialkanolaminen nahe kommt. Als Alkylenoxide können Ethylen- und Propylenoxid sowie deren Gemische (Random- oder Blockverteilung) eingesetzt werden. Die Reaktion wird üblicherweise bei Temperaturen im Bereich von 100 bis 180°C durchgeführt. Wegen des empfindlichen Zuckeranteils ist es von Vorteil, bei möglichst niedrigen Temperaturen beispielsweise im Bereich von 110 bis 140°C zu arbeiten und dafür eine etwas längere Reaktionszeit in Kauf zu nehmen. Durch den Einbau von im Durchschnitt 1 bis 10 Mol Alkylenoxid pro Mol Ester wird die Hydrophilie der Sorbitolesterquats gesteigert, die Löslichkeit verbessert und die Reaktivität gegenüber anionischen Tensiden herabgesetzt.

### Quaternierung und Alkylierungsmittel

Die Quaternierung der Fettsäure/Dicarbonsäure-trialkanolamin/sorbitolester kann in an sich bekannter Weise durchgeführt werden. Obschon die Umsetzung mit den Alkylierungsmitteln auch in Abwesenheit von Lösungsmitteln durchgeführt werden kann, empfiehlt sich die Mitverwendung zumindest von geringen Mengen Wasser oder niederen Alkoholen, vorzugsweise Isopropylalkohol, zur Herstellung von Konzentraten, die einen Feststoffanteil von mindestens 85 und insbesondere mindestens 90 Gew.-% aufweisen.

Als Alkylierungsmittel kommen Alkylhalogenide wie beispielsweise Methylchlorid, Dialkylsulfate wie beispielsweise Dimethylsulfat oder Diethylsulfat, Dialkylcarbonate wie beispielsweise Dimethylcarbonat oder Diethylcarbonat in Frage.

Üblicherweise werden die Ester und die Alkylierungsmittel im molaren Verhältnis 1 : 0,95 bis 1 : 1,05, also annähernd stöchiometrisch eingesetzt. Die Reaktionstemperatur liegt gewöhn-lich bei 40 bis 80 und insbesondere bei 50 bis 60°C. Im Anschluß an die Reaktion empfiehlt es sich, nichtumgesetztes Alkylierungsmittel durch Zugabe beispielsweise von Ammoniak, einem (Alkanol)amin, einer Aminosäure oder einem Oligopeptid zu zerstören, wie dies beispielsweise in der Deutschen Patentanmeldung **DE-A1 40 26 184** (Henkel) beschrieben wird.

### Dispergatoren und Emulgatoren

Üblicherweise wird die Quaternierung entweder wasserfrei oder in Gegenwart von geringen Mengen eines Lösungsmittels (z.B. Isopropylalkohol) durchgeführt. Je nach Einsatzzweck der Sorbitolesterquats kann es jedoch vorteilhaft sein, einen zukünftig mitzuverwendenen Dispergator oder Emulgator in das Reaktionsprodukt einzubauen, d.h. auf das eigentliche Lösungsmittel zu verzichten, das ja eigentlich nur dazu dient, eine flüssige Phase herzustellen, und die Quaternierung in Gegenwart des Dispergators/Emulgators als Solvens durchzuführen. Ein entsprechendes Verfahren ist beispielsweise in den Deutschen Patentschriften **DE-C1 43 08 794, DE-C1 43 35 782** und **DE-C1 43 39 643** (Henkel) beschrieben.

Als Dispergatoren und/oder Emulgatoren kommen beispielsweise **Fettalkohole** in Betracht. Typische Vertreter sind Capronalkohol, Caprylalkohol, 2-Ethylherylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen wie beispielsweise Kokos-, Palm-, Palmkern- oder Talgfettalkohol.

Weiterhin sind als Dispergatoren und/oder Emulgatoren **Polyole** geeignet. Typische Vertreter sind
- Glycerin;
- Alkylenglycole wie beispielsweise Ethylenglycol, Diethylenglycol, Propylenglycol;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Als weitere Gruppe von geeigneten Dispergatoren und/oder Emulgatoren sind Partialglyceride wie z.B. Mono- und/oder Diglyceride sowie anionische und nichtionische Tenside zu nennen. Unter den nichtionischen Tensiden ist der Einsatz von Alkyloligoglucosiden, Fettsäure-N-alkylglucamiden und/oder Anlagerungsprodukten von durchschnittlich 1 bis 50 Mol Ethylenoxid an die oben genannten Fettalkohole besonders bevorzugt. Das Gewichtsverhältnis Ester zu Dispergator/Emulgator kann im Bereich 30 : 70 bis 70 : 30 liegen.

### Tenside

Die erfindungsgemäßen Sorbitolesterquats können zusammen mit weiteren anionischen, nichtionischen, kationischen und/oder amphoteren Tensiden eingesetzt werden. Wegen des Problems der Adduktbildung zwischen kationischen und anionischen Tensiden sind natürlich Mischungen der Sorbitolesterquats mit nichtionischen, amphoteren und zwitterionischen Tensiden bevorzugt. Sorbitolesterquats, insbesondere solche, die über Polyoxyalkylengruppen verfügen, besitzen jedoch gegenüber anionischen Tensiden eine vergleichsweise stark herabgesetzte Reaktivität, so daß das Problem der Salzbildung und/oder Inaktivierung in der Praxis kaum zum Tragen kommt.

Typische Beispiele für **anionische Tenside** sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischether-sulfate, Monoglycerid(ether)sulfate, Fettsäureamid(ether)sulfate, Mono- und Dialkylsul-fosuccinate, Mono- und Dialkylsulfosuccinamate, Sulfotriglyceride, Amidseifen, Ether-carbonsäuren und deren Salze, Fettsäureisethionate, Fettsäuresarcosinate, Fettsäuretauride, Acyllactylate, Acyltartrate, Acylglutamate, Acylaspartate, Alkyloligoglucosidsulfate, Protein-fettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Typische Beispiele für **nichtionische Tenside** sind Fettalkoholpolyglycolether, Alkylphenolpolyglycolether, Fettsäurepolyglycolester, Fettsäureamidpolyglycolether, Fettaminpolygly-colether, alkoxylierte Triglyceride, Mischether bzw. Mischformale, Alk(en)yloligoglykoside, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Typische Beispiele für **kationische Tenside** sind quartäre Ammoniumverbindungen und Esterquats, insbesondere quaternierte Fettsäuretrialkanolaminester-Salze.

Typische Beispiele für **amphotere bzw. zwitterionische Tenside** sind Alkylbetaine, Alkylamidobetaine, Aminopropionate, Aminoglycinate, Imidazoliniumbetaine und Sulfobetaine.

Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Ber-in, 1987, S. 54-124** oder **J.Falbe (ed.), "Katalysatoren, Tenside und Mineralöladditive", Thieme Verlag, Stuttgart, 1978, S. 123-21**7 verwiesen. Aus anwendungstechnischer Sicht sind Abmischungen von Sorbitolesterquats mit den genannten Tensiden im Gewichtsverhältnis 10 : 90 bis 90 : 10 bevorzugt. Besonders vorteilhafte Eigenschaften werden bei Kombinationen von Sorbitolesterquats mit Alkyloligoglucosiden, Fettsäure-N-alkylglucamiden und/oder Betainen erhalten.

### Gewerbliche Anwendbarkeit

Die erfindungsgemäßen Sorbitolesterquats weisen ein ausgezeichnetes Avivagevermögen auf, vermindern die elektrostatische Aufladung zwischen synthetischen und natürlichen Fasern, auch Keratinfasern, und zeichnen sich durch besonders vorteilhafte ökotoxikologische Verträglichkeit aus.

Ein weiterer Gegenstand der Erfindung betrifft daher ihre Verwendung zur Herstellung von oberflächenaktiven Mitteln wie z.B. Avivagemitteln sowie vorzugsweise Mitteln zur Haar- und Körperpflege, in denen sie in Mengen von 1 bis 50, vorzugsweise 3 bis 35 Gew.-% - bezogen auf die Mittel - enthalten sein können.

Die oberflächenaktiven Mittel, bei denen es sich vorzugsweise um Haar- und Hautpflegepräparate handelt, können ferner als weitere Hilfs- und Zusatzstoffe Ölkörper, Emulgatoren, Überfettungsmittel, Verdickungsmittel, Kationpolymere, Siliconverbindungen, biogene Wirkstoffe, Filmbildner, Konservierungsmittel, Farb- und Duftstoffe enthalten.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehr-wertigen Alkoholen (wie z.B. Dimerdiol oder Trimerdiol) und/oder Guerbetalkoholen, Trigly- ceride auf Basis C₆-C₁₀-Fettsäuren, pflanzliche Öle, verzweigte primäre Alkohole, substi-tuierte Cyclohexane, Guerbetcarbonate, Dialkylether und/oder aliphatische bzw. naphthe-nische Kohlenwasserstoffe in Betracht.

Als **Emulgatoren bzw. Co-Emulgatoren** können nichtionogene, ampholytische und/oder zwitterionische grenzflächenaktive Verbindungen verwendet werden, die sich durch eine lipophile, bevorzugt lineare Alkyl- oder Alkenylgruppe und mindestens eine hydrophile Gruppe auszeichnen. Diese hydrophile Gruppe kann sowohl eine ionogene als auch eine nichtionogene Gruppe sein. Nichtionogene Emulgatoren enthalten als hydrophile Gruppe z. B. eine Polyolgruppe, eine Polyalkylenglycolethergruppe oder eine Kombination aus Polyol- und Polyglycolethergruppe.

Bevorzugt sind solche Mittel, die als **O/W-Emulgatoren** nichtionogene Tenside aus mindestens einer der folgenden Gruppen enthalten: (a1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe: (a2) C_{12/18}-Fettsäuremono- und -diester von Anlagerungsprodukten von 1 bis 30 Mol Ethylenoxid an Glycerin; (a3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte; (a4) Alkylmono- und -oligoglycoside mit 8 bis 22 Kohlenstoffatomen im Alkylrest und deren ethoxylierte Analoga und (a5) Anlagerungsprodukte von 15 bis 60 Mol Ethylenoxid an Ricinusöl und/oder gehärtetes Ricinusöl; (a6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen. Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt. C_{8/18}-Alkylmono- und oligoglycoside, ihre Herstellung und ihre Verwendung als oberflächen-aktive Stoffe sind beispielsweise aus **US 3,839,318, US 3,707,535, US 3,547,828, DE-OS 19 43 689, DE-OS 20 36 472** und **DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxyl- methyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fett-säureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder - Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylammobuttersäuren, N-Alkyliminodipropionsäuren, N-Hydroxy-ethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Als **W/O-Emulgatoren** kommen in Betracht: (b1) Anlagerungsprodukte von 2 bis 15 Mol Ethylenoxid an Ricinusöl und/oder gehärtetes Ricinusöl; (b2) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose); (b3) Trialkylphosphate; (b4) Wollwachsalkohole; (b5) Polysiloxan-Polyalkyl-Polyether-Copolymere bzw. entsprechende Derivate; (b6) Mischester aus Pentaerythrit, Fettsäuren, Citronensäure und Fettalkohol gemaß **DE-PS 11 65 574** sowie (b7) Polyalkylenglycole.

Als **Überfettungsmittel** können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise Fettal-koholethoxylate mit eingeengter Homologenverteilung oder Alkyloligoglucoside sowie Elektrolyte wie Kochsalz und Ammoniumchlorid.

Geeignete **kationische Polymere** sind beispielsweise kationischen Cellulosederivate, kationischen Stärke, Copolymere von Diallylammoniumsalzen und Acrylamiden, quaternierte Vinylpyrrolidon/Vinylimidazol-Polymere wie z.B. Luviquat® (BASF AG, Ludwigshafen/FRG), Kondensationsprodukte von Polyglycolen und Aminen, quaternierte Kollagenpolypeptide wie beispielsweise Lauryldimonium hydroxypropyl hydrolyzed collagen (Lamequat®L, Grünau GmbH), Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH, Polyaminopolyamide wie z.B. beschrieben in der **FR-A 22 52 840** sowie deren vernetzte wasserlösilchen Polymere, kationische Chitinderivate wie beispielsweise quaterniertes Chitosan, gegebenenfalls mikrokristallin verteilt, kationischer Guar-Gum wie z.B. Jaguar® CBS, Jaguar® C-17, Jaguar® C-16 der Celanese/US, quaternierte Ammoniumsalz-Polymere wie z.B. Mirapol® A-15, Mirapol® AD-1, Mirapol® AZ-1 der Miranol/US.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor- und/oder alkylmodifizierte Siliconverbindungen. Unter **biogenen Wirkstoffen** sind beispielsweise Pflanzenextrakte und Vitaminkomplexe zu verstehen. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Konservierungsmittel** eignen sich beispielsweise Phenoxyethanol, Formaldehydlösung, Parabene, Pentandiol oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die tur kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen.

### Beispiele

### I. Herstellbeispiele

### Beispiel 1

In einem 1-l-Dreihalskolben mit Rührer, Innenthermometer und Destillationsaufsatz wurden 149 g (1 mol) Triethanolamin, 405 g (1,5 mol) teilgehärtete Palmfettsäure, 43,8 g (0,3 mol) Adipinsäure und 54,6 g (0,3 mol) Sorbitol sowie 0,7 g - entsprechend 0,1 Gew.-% bezogen auf die Einsatzstoffe - Natriumhypophosphit vorgelegt. Die Mischung wurde unter Durchlei-ten von Stickstoff innerhalb von 3 h auf 200°C erhitzt und weitere 2 h gerührt. Anschließend wurde der Ansatz abgekühlt, in 82 ml Isopropylalkohol gelöst, auf 60°C erhitzt und mit 120 g (0,95 mol) Dimethylsulfat versetzt. Nach Abschluß der Zugabe wurden weitere 3 h gerührt. Das Produkt wies folgende Kennzahlen auf:

| | |
|---|---|
| Aktivsubstanz | 1,0398 meq/g |
| Trockenrückstand | 90,7 Gew.-% |
| Stickstoff | 0,048 meq/g |
| Säurezahl | 11,9 |

### Beispiel 2

307 g (0,5 mol) des rohen Fettsäure/Dicarbonsäure-triethanolamin/sorbitolesters aus Beispiel 1 wurden wieder in einer Quaternierungsapparatur vorgelegt und dort bei 65°C mit 100 g Kokosfettalkohol zu einer homogenen Masse verrührt. Anschließend wurde wie in Beispiel 1 beschrieben, jedoch ohne Zusatz von Isopropylalkohol, quaterniert. Es resultierte eine beigegefärbte, wachsartige Masse, die sich leicht zu Schuppen verarbeiten ließ.

### Beispiel 3

Beispiel 2 wurde wiederholt, anstelle des Kokosfettalkohols jedoch 100 g einer 35 Gew.-%igen wäßrigen Paste eines Alkyloligoglucosids (Plantaren® APG 2000, Henkel KGaA, Düsseldorf/FRG) eingesetzt. Es resultierte eine hellbeigegefärbte Paste.

### Beispiel 4

Beispiel 2 wurde wiederholt, anstelle des Kokosfettalkohols jedoch 100 g eines Gemisches aus einem Kokosfettsäuremonoglycerid und einem Talgalkohol+40 EO-Addukt (Gewichtsverhältnis 1 : 1) eingesetzt. Es resultierte eine hellgelbgefärbte, wachsartige Masse, die sich leicht zu Schuppen verarbeiten ließ.

### Beispiele 5, 6 und 7

Beispiel 1 wurde wiederholt, anstelle der Adipinsäure jedoch jeweils 0,3 Mol Bernsteinsäure, Weinsäure oder Citronensäure eingesetzt. Es resultierte in allen drei Fällen wiederum dunkelgefärbte Pasten.

### Beispiele 8 und 9

Beispiel 1 wurde wiederholt, jedoch anstelle des Dimethylsulfats jeweils 0,95 Mol Methylchlorid bzw. Diethylcarbonat eingesetzt. Es resultierten in beiden Fällen wiederum dunkelgefärbte Pasten.

### Beispiel 10

Beispiel 1 wurde wiederholt, 307 g (0,5 mol) des rohen Fettsäure/Dicarbonsäure-triethanolamin/sorbitolesters in einen Autoklaven überführt und mit 5 g - entsprechend 1,8 Gew.-% bezogen auf den Ester - Natriummethylat in Form einer 30 Gew.-%igen methanolischen Lösung versetzt. Der Autoklav wurde dreimal abwechselnd evakuiert und wieder mit Stickstoff gespült, auf 125°C erhitzt und dann portionsweise mit 44 g (1 mol) Ethylenoxid beaufschlagt. Nach dem Ende der Zugabe ließ man weitere 30 min nachrühren, dann wurde der Autoklav abgekühlt und entspannt. Der ethoxylierte Triethanolaminester wurde anschließend wie in Beispiel 1 beschrieben quaterniert. Es wurde eine hellgelbgefärbte, fließfähige Paste erhalten.

### II. Anwendungsbeispiele Kosmetik

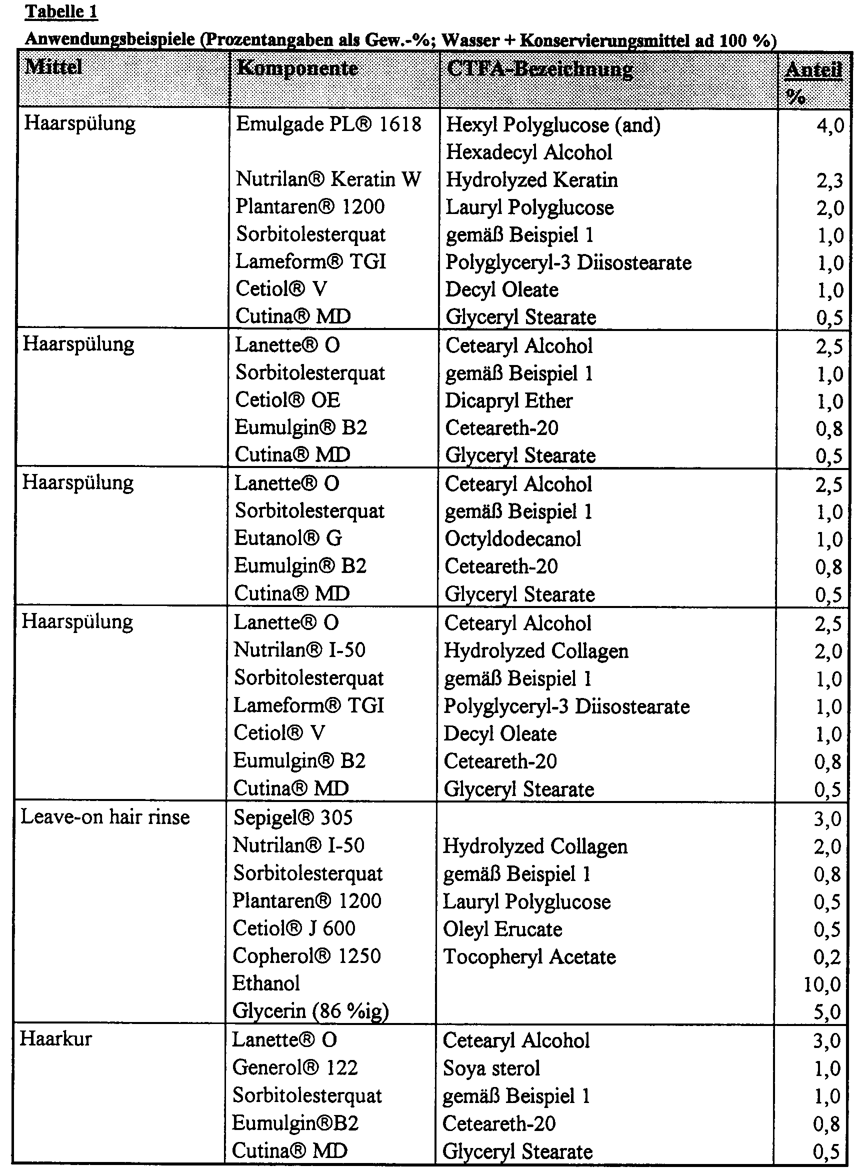

**Tabelle 1B**

| **Anwendungsbeispiele Kosmetik (Forts.)** | | | |
|---|---|---|---|
| **Mittel** | **Komponente** | **CTFA-Bezeichnung** | **Anteil** **%** |
| Haarkur | Lanette® O | Cetearyl Alcohol | 2,5 |
| | Sorbitolesterquat | gemäß Beispiel 1 | 1,5 |
| | Eumulgin® B2 | Ceteareth-20 | 1,0 |
| | Generol® 122 | Soya sterol | 1,0 |
| | Eutanol® G | Octyldodecanol | 1,0 |
| | Cutina® MD | Glyceryl Stearate | 0,5 |
| Duschbad | Texapon® K 14 S | Sodium Myreth Sulfate | 38,0 |
| | Plantaren® 2000 | Decyl Polyglucose | 7,0 |
| | Lamesoft® LMG | Glyceryl Laurate (and) Potassium Cocoyl Hydrol. Collagen | 3,0 |
| | Arylpon® F | Laureth-2 | 3,0 |
| | Sorbitolesterquat | gemäß Beispiel 1 | 0,5 |
| Duschbad | Texapon® NSO | Sodium Laureth Sulfate | 38,0 |
| | Plantaren® 2000 | Decyl Polyglucose | 7,0 |
| | Euperlan® PK 3000 | Glycol Distearate (and) Cocoamidopropyl Betaine | 3,0 |
| | Arlypon® F | Laureth-2 | 3,0 |
| | Lamesoft® LMG | Glyceryl Laurate (and) Potassium Cocoyl Hydrol. Collagen | 2,0 |
| | Sorbitolesterquat | gemäß Beispiel 1 | 0,5 |
| | Kochsalz | | 1,5 |
| Duschgel | Texapon® NSO | Sodium Laureth Sulfate | 25,0 |
| | Texapon® SB3 | Disodium Laurethsulfosuccinate | 10,0 |
| | Dehyton® K | Cocoamidopropyl Betaine | 10,0 |
| | Plantaren® 2000 | Decyl Polyglucose | 6,0 |
| | Euperlan® PK 3000 | Glycol Distearate (and) Cocoamidopropyl Betaine | 5,0 |
| | Lamesoft® LMG | Glycol Distearate (and) Cocoamidopropyl Betaine | 4,0 |
| | Cetiol® HE | PEG-7 Glyceryl Cocoate | 1,0 |
| | Arlypon® F | Laureth-2 | 1,0 |
| | Sorbitolesterquats | gemäß Beispiel 1 | 0,5 |
| Waschlotion | Plantaren® PS 10 | Sodium Laureth Sulfate (and) Lauryl Polyglucose | 16,0 |
| | Euperlan® PK 3000 | Glycol Distearate (and) Cocoamidopropyl Betaine | 5,0 |
| | Sorbitolesterquats | gemäß Beispiel 1 | 0,5 |
| | Kochsalz | | 1,5 |

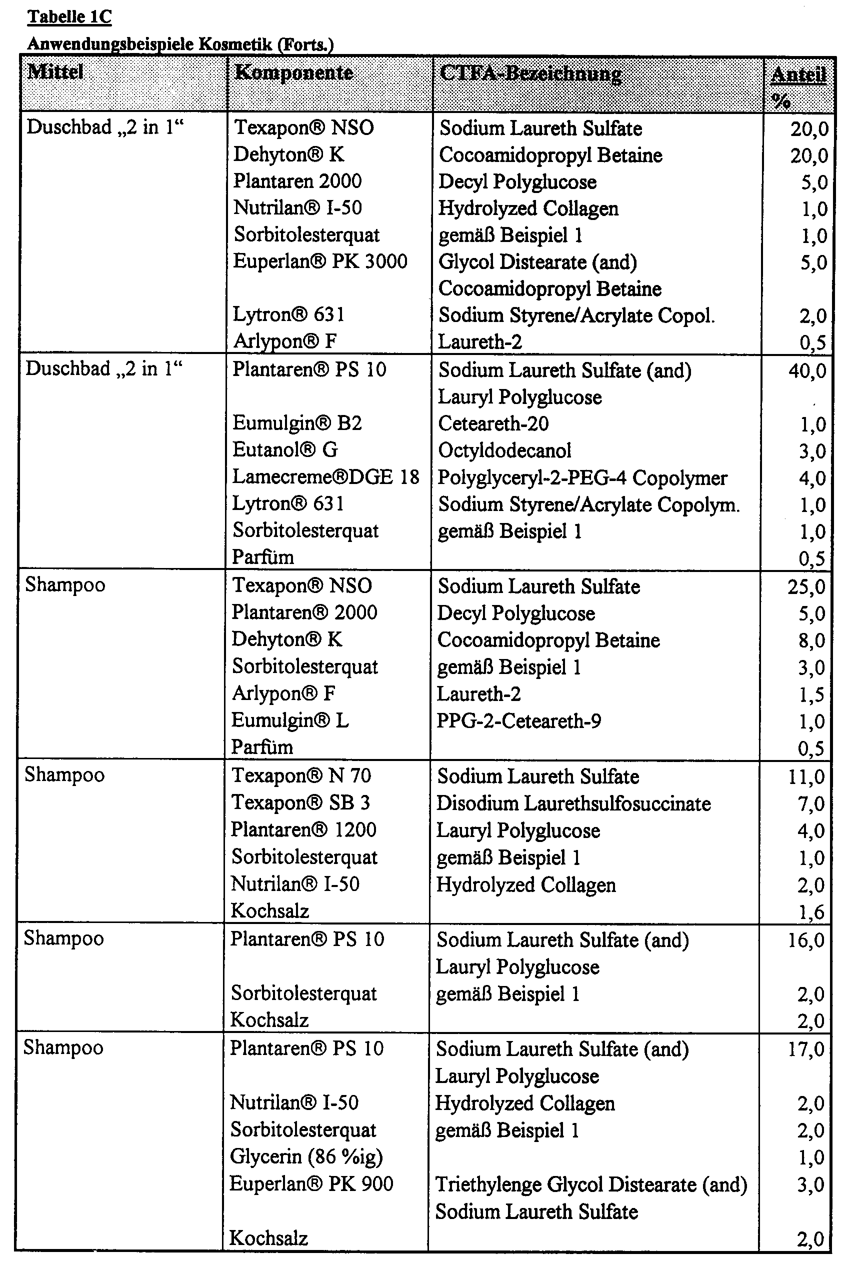

**Tabelle 1D**

| **Anwendungsbeispiele Kosmetik (Forts.)** | | | |
|---|---|---|---|
| **Mittel** | **Komponente** | **CTFA-Bezeichnung** | **Anteil** **%** |
| Shampoo | Texapon® ALS | Ammonium Laureth Sulfate | 23,0 |
| | Plantaren® 2000 | Decyl Polyglucose | 4,0 |
| | Dehyton® K | Cocoamidopropyl Betaine | 7,0 |
| | Sorbitolesterquat | gemäß Beispiel 1 | 2,0 |
| | Lamesoft® 156 | Hydrogenated Tallow Glycerides | 5,0 |
| | Monomuls® 90-L 12 | Glyceryl Laurate | 1,0 |
| | Kochsalz | | 3,0 |
| Schaumbad | Plantaren® PS 10 | Sodium Laureth Sulfate (and) Lauryl Polyglucose | 22,0 |
| | Dehyton® K | Cocoamidopropyl Betaine | 15,0 |
| | Sorbitolesterquat | gemäß Beispiel 1 | 3,0 |
| | Cetiol® HE | PEG-7 Glyceryl Cocoate | 2,0 |
| | Euperlan® PK 3000 | Glycol Distearate (and) Cocoamidopropyl Betaine | 5,0 |
| Schaumbad | Texapon® NSO | Sodium Laureth Sulfate | 30,0 |
| | Dehyton® K | Cocoamidopropyl Betaine | 10,0 |
| | Plantaren® 1200 | Lauryl Polyglucose | 10,0 |
| | Lamesoft® LMG | Glyceryl Laurate (and) Potassium Cocoyl Hydrol. Collagen | 4,0 |
| | Sorbitolesterquat | gemäß Beispiel 1 | 2,0 |
| | Guadin® AGP | Hydrolyzed Wheat Protein | 0,5 |
| Schaumbad | Melissenöl | | 5,0 |
| | Eumulgin® L | PPG-2-Ceteareth-9 | 15,0 |
| | Plantaren® 2000 | Decyl Polyglucose | 30,0 |
| | Dehyton® K | Cocoamidopropyl Betaine | 10,0 |
| | Sorbitolesterquat | gemäß Beispiel 1 | 4,0 |
| | Propylenglycol | | 4,0 |
| | Arlypon® F | Laureth-2 | 1,5 |

## Patentansprüche

1. Sorbitolesterquats, dadurch erhältlich, daß man Trialkanolamine mit einer Mischung aus Fettsäuren, Dicarbonsäuren und Sorbitol umsetzt, die resultierenden Ester gegebenenfalls alkoxyliert und anschließend in an sich bekannter Weise quaterniert.

2. Verfahren zur Herstellung von Sorbitolesterquats, bei dem man Trialkanolamine mit einer Mischung aus Fettsäuren, Dicarbonsäuren und Sorbitol umsetzt und die resultierenden Ester gegebenenfalls nach Alkoxylierung anschließend in an sich bekannter Weise quaterniert.

3. Verfahren nach Anspruch 2, **dadurch gekennzeichnet**, daß man Triethanolamin einsetzt.

4. Verfahren nach den Ansprüchen 2 und 3, **dadurch gekennzeichnet**, daß man Fettsäuren der Formel **(I)** einsetzt, 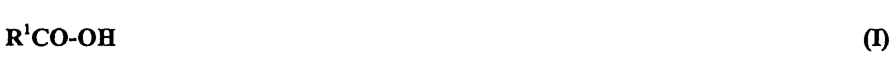 in der R¹CO für einen aliphatischen, linearen oder verzweigten Acylrest mit 6 bis 22 Kohlenstoffatomen und 0 und/oder 1, 2 oder 3 Doppelbindungen steht.

5. Verfahren nach den Ansprüchen 2 bis 4, **dadurch gekennzeichnet**, daß man Dicarbonsäuren der Formel **(II)** einsetzt, 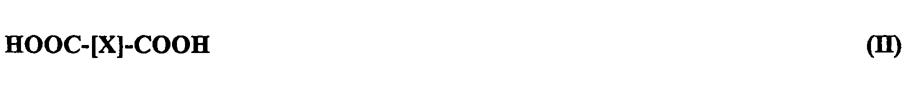 in der X für eine gegebenenfalls hydroxy- oder carboxysubstituierte Alkylengruppe mit 2 bis 12 Kohlenstoffatomen oder einen Phenylrest steht.

6. Verfahren nach den Ansprüchen 2 bis 5, **dadurch gekennzeichnet**, daß man die Fettsäuren und die Dicarbonsäuren im molaren Verhältnis 1 : 10 bis 10 : 1 einsetzt.

7. Verfahren nach den Ansprüchen 2 bis 6, **dadurch gekennzeichnet**, daß man die Trialkanolamine einerseits und die Summe aus Fettsäuren und Dicarbonsäuren andererseits im molaren Verhältnis 1 : 1,3 bis 1 : 2,4 einsetzt.

8. Verfahren nach den Ansprüchen 2 bis 7, **dadurch gekennzeichnet**, daß man die Dicarbonsäuren und das Sorbitol im molaren Verhältnis 1 : 0,75 bis 1 : 1,25 einsetzt.

9. Verfahren nach den Ansprüchen 2 bis 8, **dadurch gekennzeichnet**, daß man die Veresterung in Gegenwart von hypophosphoriger Säure bzw. deren Alkalisalzen durchführt.

10. Verfahren nach den Ansprüchen 2 bis 9, **dadurch gekennzeichnet**, daß man Alkylierungsmittel einsetzt, die ausgewählt sind aus der Gruppe, die gebildet wird von Alkylhalogeniden, Dialkylsulfaten und Dialkylcarbonaten.

11. Verfahren nach den Ansprüchen 2 bis 10, **dadurch gekennzeichnet**, daß man die Quaternierung in Gegenwart von Dispergatoren bzw. Emulgatoren durchführt, die ausgewählt sind aus der Gruppe, die gebildet wird von Fettalkoholen, Polyolen, Partialglyceriden, anionischen Tensiden und nichtionischen Tensiden.

12. Verwendung von Sorbitolesterquats nach Anspruch 1 zur Herstellung von oberflächenaktiven Mitteln.
